# EUROPEAN PATENT SPECIFICATION

(11) **EP 2 341 772 B1**
(45) Date of publication and mention of the grant of the patent: **17.04.2013**
(21) Application number: 09818570.5
(22) Date of filing: 02.10.2009
(51) Int. Cl.: C07H 13/08, C07H 19/12, A01N 43/04, A61K 31/70

(54) **SYNTHESIS OF DECITABINE**
DECITABINSYNTHESE
SYNTHÈSE DE DÉCITABINE

(30) Priority: 03.10.2008 US 102571 P
(43) Date of publication of application: 13.07.2011
(73) Proprietor: ScinoPharm Taiwan Ltd., Tainan County 741 (TW)
(72) Inventor: HENSCHKE, Julian, Paul, Summertown South Australia 5141 (AU); ZHANG, Xiaoheng, Guan Nan County Jiangsu Province (CN); YU, Jianbo, Zhejiang Province (CN); HU, Kun, Heilongjiang Province (CN); MEI, Lijun, Jiangxi Province (CN)
(74) Representative: Gulde Hengelhaupt Ziebig & Schneider
(86) International application number: PCT/US2009/059385
(87) International publication number: WO 2010/040056

(56) References cited:
- EP-A1- 2 048 151
- WO-A1-2009/086687
- US-A- 5 366 987
- US-A1- 2004 266 996
- US-A1- 2006 205 687

## Description

### BACKGROUND OF THE INVENTION

This invention relates to the synthesis of Decitabine (also known as 2'-deoxy-5-azacytidine; 5-aza-2'-deoxycytidine; DAC; 4-amino-1-(2-deoxy-β-D-erythro-pentofuranosyl)-1,3,5-triazin-2(1*H*)-one) API (active pharmaceutical ingredient) which once formulated is used in injectable form for the treatment of myelodysplastic syndromes (MDS). Furthermore, the use of Decitabine in other diseases such as AML, CML, stem cell transplant, sickle cell anemia and thalassemia is under investigation.

The present invention is a process for the synthesis of Decitabine. Specifically, in one embodiment the invention is related to a set of reaction conditions that can be utilised for the synthesis of a precursor of Decitabine **(p-Cl-Bz-IM4)** which itself has a certain quality that makes it useful for the synthesis of Decitabine in higher yields than that that can be achieved using prior art literature methods that utilise related synthetic methodology. The process of the invention provides the drug substance Decitabine suitable for human use which is free of metallic residues, which is a failing of other published methods, and is industrially economical.

One advantage of this invention over others in the literature (*e.g.*, J. Org. Chem., 1974, 39, 3672-3674, J. Org. Chem., 1986, 51, 3211-3213 and EP 2048151 A) for preparation of the ultimate precursor of Decitabine **(p-Cl-Bz-IM4)** is the use of the non-metallic Lewis acid TMS-triflate or the Brønsted acid triflic acid instead of tin tetrachloride, which itself is toxic, in the key carbohydrate (protected 2-deoxy-ribose derivative) and base (protected 5-azacytosine) coupling step. Use of tin tetrachloride, which is the standard Lewis acid in this type of coupling process can lead to the final drug substance being contaminated with tin residues and leads to difficulty in disposing waste streams. Another advantage of this invention over another Decitabine process in the literature (J. Org. Chem., 1970, 35, 491-495) is that the use of these catalysts allows the key carbohydrate and base coupling step reaction to be performed in a shorter period of time. Furthermore, the process also does not require the use of column chromatography, preparative thin layer chromatography or fractional crystallisation for purification to obtain the product drug substance or intermediates.

There are several reasons we didn't develop a process based on the prior art described in the literature that utilises tin tetrachloride or use a completely non-catalysed system. Most significantly the use of tin tetrachloride as a coupling catalyst (for coupling of 1-halo-2-deoxyribose or 1-*O*-acetyl-2-deoxyribose systems with the base - see J. Org. Chem., 1974, 39, 3672-3674 and J. Org. Chem., 1986, 51, 3211-3213) or no catalyst at all (for 1-halo-2-deoxyribose systems - see J. Org. Chem., 1970, 35, 491-495) both lead to approximately 1:1 mixtures of the crude α- and ß-anomers of the ultimate precursor of Decitabine **(p-Cl-Bz-IM4).** Crucially we found that it is difficult to obtain good quality and a good yield of the ß-anomer of Decitabine, which is the active drug form, by deprotection of the ultimate precursor **(p-Cl-Bz-IM4)** when its anomeric ratio is close to 1:1. We found that the isolated yield (calculated based on the ß-anomer of ultimate precursor **p-Cl-Bz-IM4)** of ß-Decitabine *(i.e.,* the API) is higher, the greater the ratio of the two anomers of the crude ultimate precursor of ß-/α-Decitabine and that this is a nonlinear relationship. That is, the yield is higher and higher the more enriched the ß-anomer of protected precursor of ß-Decitabine **(p-Cl-Bz-IM4)** is, even when the yield is calculated based the quantity of the ß-anomer in the mixture. Therefore we concluded that the ratio of anomers of protected-Decitabine precursor is very important for a viable process to make Decitabine.

### SUMMARY OF THE INVENTION

In our process we consistently obtain at least a 1:2 mixture of crude α- and ß-anomer of the ultimate precursor of Decitabine **(p-Cl-Bz-IM4)** which provides a good quality and a good yield of the ß-anomer of Decitabine following the deprotection and purification steps. To achieve this we discovered that the coupling reaction must be conducted at low temperature and that the coupling catalyst TMS-triflate or TfOH must be deactivated with a base at low temperature at the end point of the reaction and before the aqueous work-up step is conducted. This is done to avoid equilibration of the protected- α- and ß-anomers of Decitabine **(p-Cl-Bz-IM4)** which occurs more and more rapidly the higher the temperature and ultimately results in a lower ratio of the two anomers. The reaction must also be conducted in a non-polar organic solvent to inhibit the otherwise significantly rapid decrease of the α- and ß-anomeric ratio of the ultimate precursor of Decitabine **(p-Cl-Bz-IM4).**

Our process can obtain API grade Decitabine economically that has a quality equal to that of the innovator of Decitabine.

Our process combines a range of different variables (temperature, use of catalyst deactivation, solvent, reaction concentration, amount of catalyst) to obtain a higher quantity of the β-anomer, than is otherwise possible using standard conditions, which is necessary for a higher yield of Decitabine.

### DETAILED DESCRIPTION OF THE INVENTION

### EXAMPLE 1 - Preparation of 3,5-di-O-(p-chlorobenzoyl)-Decitabine

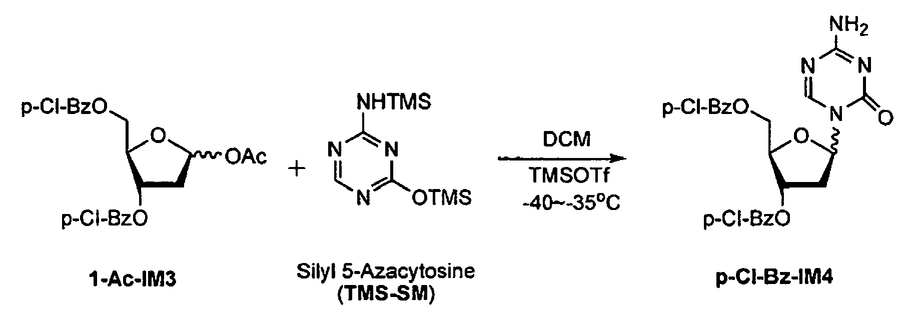

1-*O*-Acetyl-3,5-di-*O*-(*p*-chlorobenzoyl)-2-deoxyl-D-ribofuranose (500 g, 90% HPLC pure equivalent to 0.99 mol), dichloromethane DCM (5.93 Kg) and silyl 5-azacytosine (254 g, 0.99 mol) are cooled to -45 - -40°C and TMSOTf (231 g, 1.04 mol) is added and the solution is stirred at -40°C ~ -35°C for 9 h. 33% MeNH₂ in MeOH (97.6 g, 1.04 mol) is added to the reaction solution at -40°C to -35°C and it is then diluted with DCM (5.93 Kg). The solution is allowed to warm to 20~25°C and a saturated solution of NaHCO₃ (8.3 Kg) is added at 20~25°C and is stirred for 30~40 min. The organic phase is separated, dried over 4Å molecular sieves and filtered and extra DCM (3.7 Kg) is used to rinse the 4Å molecular sieves. The filtrate is combined and evaporated to dryness. The solid is dried *in vacuo* at 50°C, then ground into a fine powder, and dried again at 50°C in vacuum. 460 g Of the title compound is obtained as a 15-30%: 40-60% mixture of the α-anomer and the β-anomer as indicated by HPLC analysis.

### EXAMPLE 2 - Crude Decitabine

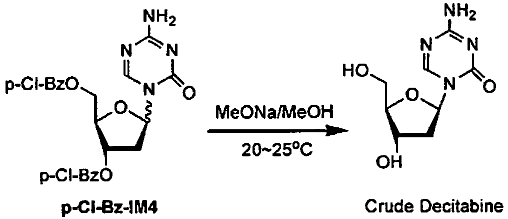

MeOH (1.8 Kg) and 3,5-di-*O*-(*p*-chlorobenzoyl)-Decitabine (455 g, 63.9% HPLC pure equivalent to 0.58 mol) are stirred at 20-25°C. A 29% MeONa in MeOH solution (42.8 g, 0.23 mol) is added to the mixture which is then stirred at 20∼25 °C for 30 min. The solid is filtered, washed three times with *n*-heptane (120 mL each) and then dried at 50°C *in vacuo* to give 42.5 g of crude Decitabine in 93.6% purity as indicated by HPLC analysis.

### EXAMPLE 3 - Purification of crude Decitabine with MeOH

Crude Decitabine (42.5 g) and MeOH (2.85 kg) are stirred and heated to reflux until the mixture almost completely dissolved. The solution is hot filtered to remove the insoluble material. The filtrate is stirred and cooled and the crystal starts to form at about 40°C. The resultant slurry is stirred for 1 h at the cloudy point and is then slowly cooled further. The slurry is stirred at 10-25°C for 4∼8 h and then filtered. The filtered cake is washed three times with MeOH (40 mL each) and dried at 50°C *in vacuo* for 8 h to give 27.5 g of pure Decitabine in 64.7% yield.

### EXAMPLE 4 - Purification of crude Decitabine with DMSO and MeOH

Crude Decitabine (1.5 g, 90.6% purity by HPLC) and anhydrous MeOH (29 mL) are stirred and heated at reflux for 30 min. DMSO (9.3 mL) is slowly added to the solution resulting in almost complete dissolution in the mixed solvent at 60~65°C. The mixture is filtered, and the filtrate is slowly cooled. Decitabine crystallises from the solution. At 4°C, the slurry is filtered and the filtered cake is washed three times with MeOH (3 mL) and dried *in vacuo* at 50°C to give dry API grade Decitabine (0.9 g, 99.82% pure as indicated by HPLC analysis).

### EXAMPLE 5 - Preparation of 3,5-di-O-(p-chlorobenzoyl)-Decitabine

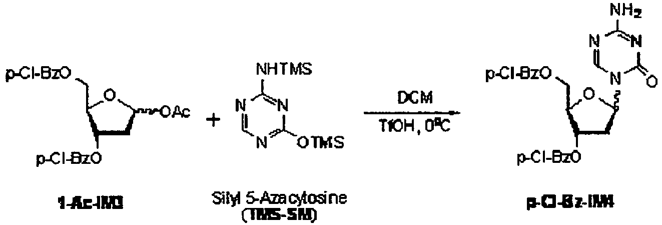

A mixture of 1-*O*-Acetyl-3,5-di-*O*-(*p*-chlorobenzoyl)-2-deoxyl-D-ribofuranose (5 g of 90% HPLC pure material which is equivalent to 9.9 mmol), DCM (50 mL) and silyl 5-azacytosine (2.5 g, 9.9 mmol) is cooled to about 0°C and TfOH (1.1 g, 6.9 mmol) is added to the solution. The solution is stirred for 5 h at about 0°C and then diluted with DCM (100 mL) followed by the addition of a saturated solution of NaHCO₃ (75 mL) at 20~25°C. The organic phase is separated, dried over anhydrous MgSO₄ and filtered. The MgSO₄ is washed with DCM (30 mL) and the filtrates are combined and evaporated to dryness at 20~40°C under reduced pressure to give 4.5 g of the title compound which was shown by HPLC analysis to be composed of 29.0% of the α-anomer and 37.4% of the β-anomer.

### EXAMPLE 6 -TfOH Catalyst the Coupling Reaction.

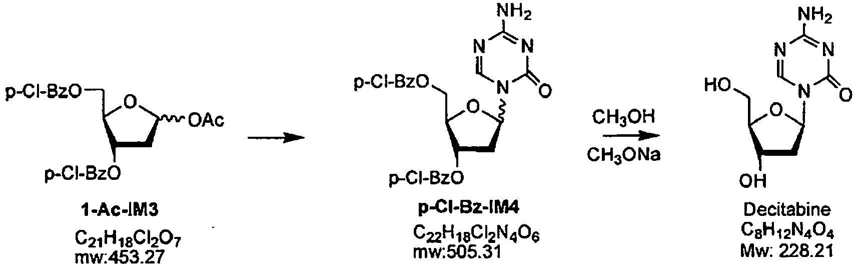

### A. Preparation of p-Cl-Bz-IM4.

**1-Ac-IM3** (20.0 g, purity: 96.0 area% by HPLC, 42.4 mmol) and **TMS-SM** (11.4 g, 44.5 mmol) were added to a dried four-necked flask followed by MeCN (300 mL, 15 P). The mixture was cooled to -15°C ~-20°C under an atmosphere of N_{2(g)}. TfOH (3.2 g, 21.3 mmol) was charged into the flask and the mixture was stirred at this temp until HPLC analysis indicated no change in the amount of **1-Ac-IM3** (about 44 h). The mixture was then stirred at between 0 to 10°C until HPLC analysis indicated that no more than 3 area% of **1-Ac-IM3** remained by HPLC analysis. The reaction mixture was diluted with DCM (600 mL, 30 P) and washed with aqueous saturated sodium bicarbonate (900 mL, 45 P) at 25°C for 15 min. The organic layer was dried using 4Å molecular sieves (180 g) at 25°C for 5 h. This was evaporate to dryness under vacuum at 40°C and then dried at 40°C for 16 h. 15.7 g of a light yellow coloured solid was obtained. HPLC analysis showed 18.5% **α-p-Cl-Bz-IM4** and 44.1% **β-p-Cl-Bz-IM4.** The yield was 45.9%.

### B. Deprotection.

To a dried 4-necked flask was added MeOH (78.5 mL, 5 P) and 29% MeONa/MeOH (1.45 g, 7.78 mmol) and the mixture was warmed to 25°C. **p-Cl-Bz-IM4** (15.7 g, purity: 62.6 area% by HPLC, 19.4 mmol) was charged into the flask and kept at 25°C for 2 h and then filtered. The filter cake was washed three times with MeOH and dried at 40°C to give white crystals of crude Decitabine (1.51 g, 45.9% yield, purity: 95.2 area% by HPLC). C. Recrystallization.

The crude Decitabine was dissolved in MeOH (128.3 mL, 85P) at reflux temperature and was then slowly cooled about 10°C/h. When solid appeared the mixture was kept at this temperature for 1 h and was then cooled to room temp (25°C) for 8 h, and then filtered. The filter cake was washed three times with MeOH and dried at 40°C to give Decitabine (0.78 g, 53.7% yield, purity: >99 area% by HPLC).

## Claims

1. A method for producing a β-enriched protected-IM4 comprising the steps of:
coupling 1-*O*-Acetyl-3,5-di-*O*-(p-chlorobenzoyl)-2-deoxy-D-ribofuranose and a protected 5-azacytosine in the presence of an organic solvent and a catalyst comprising a non-metallic Lewis acid or a Brønsted acid, and conducting the coupling reaction at a temperature of 0°C or below to produce the protected-IM4; and
quenching the coupling reaction with an organic amine at a temperature of 0°C or below.

2. The method of claim 1 wherein the coupling reaction is conducted at a temperature of 0 to -80°C

3. The method of claim 1 wherein the coupling reaction is conducted at a temperature of about -40°C.

4. The method of claim 1 wherein the protected-IM4 is p-Cl-Bz-IM4.

5. The method of claim 1 wherein the non-metallic Lewis acid is trimethylsilyltrifluoromethylsulfonate (TMSOTf).

6. The method of claim 1 wherein the Brønsted acid is triflic acid (TfOH).

7. The method of claim 1 wherein the organic amine is MeNH₂ or EtNH₂.

8. The method of claim 1 wherein the organic solvent is non-polar.

9. The method of claim 8 wherein the organic solvent is DCM.

10. The method of claim 1 further comprising the step of drying the protected-IM4 after the quenching step.

11. The method of claim 1 further comprising the step of deprotecting the protected-IM4 to produce Decitabine.

12. The method of claim 11 wherein the step of deprotecting the protected-IM4 is in the presence of an alkoxide.

13. The method of claim 12 wherein the alkoxide is sodium methoxide.

14. The method of claim 11 further comprising the step of washing the Decitabine with an organic solvent.

15. The method of claim 11 further comprising the step of recrystallizing the Decitabine in an alcohol solution or an alcohol and DMSO mixture.

16. The method of claim 15 wherein the alcohol is methanol.

17. The method of claim 1 wherein the organic amine is a primary amine.

## Patentansprüche

1. Verfahren zum Herstellen eines β-angereicherten geschützten IM4, das die folgenden Schritte umfasst:
Koppeln von 1-O-Acetyl-3,5-di-O-(p-chlorbenzoyl)-2-desoxy-D-ribofuranose und eines geschützten 5-Azacytosins in Gegenwart eines organischen Lösungsmittels und eines Katalysators, umfassend eine nicht-metallische Lewis-Säure oder eine Br⌀nstedt-Säure, und Durchführen der Kopplungsreaktion bei einer Temperatur von 0°C oder darunter zur Herstellung des geschützten IM4; und
Abschrecken (Quenching) der Kopplungsreaktion mit einem organischen Amin bei einer Temperatur von 0°C oder darunter.

2. Verfahren nach Anspruch 1, wobei die Kopplungsreaktion bei einer Temperatur von 0 bis -80°C durchgeführt wird.

3. Verfahren nach Anspruch 1, wobei die Kopplungsreaktion bei einer Temperatur von etwa -40°C durchgeführt wird.

4. Verfahren nach Anspruch 1, wobei es sich bei dem geschützten IM4 um p-Cl-Bz-IM4 handelt.

5. Verfahren nach Anspruch 1, wobei es sich bei der nicht-metallischen Lewis-Säure um Trimethylsilyltrifluormethylsulfonat (TMSOTf) handelt.

6. Verfahren nach Anspruch 1, wobei es sich bei der Brs⌀nstedt-Säure um Trifluormethansulfonsäure (TfOH) handelt.

7. Verfahren nach Anspruch 1, wobei es sich bei dem organischen Amin um MeNH₂ oder EtNH₂ handelt.

8. Verfahren nach Anspruch 1, wobei das organische Lösungsmittel unpolar ist.

9. Verfahren nach Anspruch 8, wobei es sich bei dem organischen Lösungsmittel um DCM handelt.

10. Verfahren nach Anspruch 1, welches ferner den Schritt des Trocknens des geschützten IM4 nach dem Abschreckungsschritt (Quenching-Schritt) umfasst.

11. Verfahren nach Anspruch 1, welches ferner den Schritt des Entschützens des geschützten IM4 zur Herstellung von Decitabin umfasst.

12. Verfahren nach Anspruch 11, wobei der Schritt des Entschützens des geschützten IM4 in Gegenwart eines Alkoxids stattfindet.

13. Verfahren nach Anspruch 12, wobei es sich bei dem Alkoxid um Natriummethoxid handelt.

14. Verfahren nach Anspruch 11, welches ferner den Schritt des Waschens des Decitabins mit einem organischen Lösungsmittel umfasst.

15. Verfahren nach Anspruch 11, welches ferner den Schritt des Umkristallisierens des Decitabins in einer Alkohollösung oder einem Gemisch aus Alkohol und DMSO umfasst.

16. Verfahren nach Anspruch 15, wobei es sich bei dem Alkohol um Methanol handelt.

17. Verfahren nach Anspruch 1, wobei es sich bei dem organischen Amin um ein primäres Amin handelt.

## Revendications

1. Procédé de production d'un IM4 protégé β-enrichi comprenant les étapes consistant à :
coupler la 1-O-acétyl-3,5-di-O-(p-chlorobenzoyl)-2-désoxy-D-ribofuranose et une 5-azacytosine protégée en présence d'un solvant organique et d'un catalyseur comprenant un acide de Lewis non métallique ou un acide de Br⌀nsted, et réaliser la réaction de couplage à une température inférieure ou égale à 0 °C pour produire un IM4 protégé ; et
neutraliser la réaction de couplage avec une amine organique à une température inférieure ou égale à 0 °C.

2. Procédé selon la revendication 1, dans lequel la réaction de couplage est réalisée à une température de 0 à -80 °C.

3. Procédé selon la revendication 1, dans lequel la réaction de couplage est réalisée à une température d'environ -40 °C.

4. Procédé selon la revendication 1, dans lequel l'IM4 protégé est le p-Cl-Bz-IM4.

5. Procédé selon la revendication 1, dans lequel l'acide de Lewis non métallique est le triméthylsilyltrifluorométhylsulfonate (TMSOTf).

6. Procédé selon la revendication 1, dans lequel l'acide de Br⌀nsted est l'acide triflique (TfOH).

7. Procédé selon la revendication 1, dans lequel l'amine organique est la MeNH₂ ou l'EtNH₂.

8. Procédé selon la revendication 1, dans lequel le solvant organique est non polaire.

9. Procédé selon la revendication 8, dans lequel le solvant organique est le DCM.

10. Procédé selon la revendication 1, comprenant en outre l'étape consistant à sécher l'IM4 protégé après l'étape de neutralisation.

11. Procédé selon la revendication 1, comprenant en outre l'étape consistant à déprotéger l'IM4 protégé pour produire de la Décitabine.

12. Procédé selon la revendication 11, dans lequel l'étape consistant à déprotéger l'IM4 protégé est réalisée en présence d'un alcoxyde.

13. Procédé selon la revendication 12, dans lequel l'alcoxyde est le méthoxyde de sodium.

14. Procédé selon la revendication 11, comprenant en outre l'étape consistant à laver la décitabine avec un solvant organique.

15. Procédé selon la revendication 11, comprenant en outre l'étape consistant à recristalliser la décitabine dans une solution d'un alcool ou un mélange d'un alcool et de DMSO.

16. Procédé selon la revendication 15, dans lequel l'alcool est le méthanol.

17. Procédé selon la revendication 1, dans lequel l'amine organique est une amine primaire.
